# EUROPEAN PATENT SPECIFICATION

(11) **EP 3 664 693 B1**
(45) Date of publication and mention of the grant of the patent: **12.06.2024**
(21) Application number: 18779151.2
(22) Date of filing: 10.09.2018
(51) Int. Cl.: A61B 5/00, A61B 8/12

(54) **IMAGING SYSTEM AND METHOD THEREFOR**
BILDGEBUNGSSYSTEM UND VERFAHREN DAFÜR
SYSTÈME D'IMAGERIE ET PROCÉDÉ ASSOCIÉ

(30) Priority: 12.09.2017 US 201715701570
(43) Date of publication of application: 17.06.2020
(73) Proprietor: Colgate-Palmolive Company, New York, NY 10022 (US)
(72) Inventor: SUBHASH, Hrebesh Molly, Somerset, NJ 08873 (US)
(74) Representative: Mooser, Sebastian Thomas
(86) International application number: PCT/US2018/050160
(87) International publication number: WO 2019/055332

(56) References cited:
- WO-A1-2014/144278
- US-A1- 2013 289 381
- US-A1- 2013 289 381
- US-A1- 2014 221 842
- US-A1- 2014 221 842
- LIN C.Y. ET AL: "Photoacoustic Imaging for Noninvasive Periodontal Probing Depth Measurements", JOURNAL OF DENTAL RESEARCH, vol. 97, no. 1, 7 September 2017 (2017-09-07), pages 23-30, XP055843205, US ISSN: 0022-0345, DOI: 10.1177/0022034517729820
- LIN C.Y. ET AL: "Photoacoustic Imaging for Noninvasive Periodontal Probing Depth Measurements", JOURNAL OF DENTAL RESEARCH, vol. 97, no. 1, 7 September 2017 (2017-09-07), pages 23-30, XP055843205, US ISSN: 0022-0345, DOI: 10.1177/0022034517729820

## Description

### BACKGROUND

Oral health problems can take many forms, such as tooth decay, oral cancer, periodontal disease, and bad breath. While some health problems manifest on the surface of oral tissue, others are subsurface. Currently, there are no reliable clinical imaging systems which can provide indicators of oral health problems in a quantitative depth resolved manner.

Optical coherence tomography is one imaging technology which has shown promise for a wide range of oral diagnostic applications. However, the imaging depth of optical coherence tomography is limited to 1 to 2 mm and it is incapable of providing any spectroscopic information at spectral wavelengths which penetrate deeper into tissue. Intraoral fluorescence and cross-polarization imaging are another imaging modality which has shown promise by providing surface images of oral tissue in real time. This technology is, however, incapable of providing depth resolved or spectroscopic information of imaged tissue. Ultrasonography (US) is another imaging technology which has gained more use for diagnosing oral tissue. This technology has the advantage of being able to produce cross sectional images of tissue at varying depths, which is useful for detecting and diagnosing subsurface diseases and health problems. Ultrasonography can also produce ultrasound Doppler images, which show vascular flow for differentiating normal tissue from tissue showing signs of disease. However, traditional ultrasonography can only provide limited spatial resolution in deep tissues, and the contrast it provides between tissue structures can be limited. Photoacoustic imaging (PAI) is one of the more recent imaging technologies that has been used for oral tissue diagnostics. PAI has the advantage that it combines the high spectroscopic based contrast of optical imaging with high spatial resolution, and it is capable of providing subsurface imaging combined with information about tissue function. WO 2014/144 278 A1 discloses an optoacoustic device designed to be applied to the surface of a user's skin for detecting a tumor or lesion in internal body structures of the user.

None of these imaging technologies alone are sufficient for diagnosing tissue health, both one the surface and sub-surface, and particularly with the range of tissue structure and potential issues that may manifest sub-surface. An imaging technology is therefore desirable that includes several of the advantages found in multiple ones of the aforementioned imaging technologies. Such an imaging technology should also be cost-effective, compact, and easy to use, such that it can readily be used for point-of-care diagnostic applications. In addition, such an imaging technology should enable the rapid and accurate diagnosis and monitoring of patients, while also reducing the cost and time associated with healthcare services.

US 2013/289381 A1 discloses an imaging system that provides ultrasonic imaging and optoacoustic imaging co-registered through application of a same hand-held probe to generate and detect ultrasonic and optoacoustic signals. US 2014/221842 A1 discloses a photoacoustic device capable of performing both intravascular photoacoustic imaging and intravascular ultrasound imaging.

### BRIEF SUMMARY

Exemplary embodiments according to the present disclosure are directed to imaging systems and methods which employ photoacoustic imaging (PAI) to image tissue. The imaging system includes a miniature hand-held imaging probe coupled to a data processing and display unit. The system may also incorporate an ultrasound transducer as part of the probe, thereby enabling both photoacoustic images and ultrasound images (B-mode and/or Doppler) to be processed at the same time. The images obtained from the different modalities may be displayed in a co-registered manner so that relationships may be seen between structures and features of the different images. In addition, both individual and co-registered images may be displayed as a video. The imaging method includes positioning the probe head adjacent tissue to be imaged, obtaining the desired images by actuating at least the light source used to obtain photoacoustic images, and then processing the data signal generated by the probe to produce one or more images of the tissue. By also actuating the ultrasound transducer using a common timing signal that is also used to actuate the light source, data for both photoacoustic images and ultrasound images may be generated using a single probe. The imaging method may also include using a display device to display the one or more images in real time.

In one aspect, the invention can be an imaging system according to claim 1.

In another aspect, the invention can be an imaging method according to claim 9.

An example useful for understanding the present invention can be an imaging system including: a probe head including: a light source for emitting light in at least one spectral waveband suitable to generate a photoacoustic response in tissue, the light source comprising at least two light emitting elements, each light emitting element comprising an emission plane; and an ultrasound receiver comprising a receiver plane, wherein each emission plane is positioned at an acute angle with respect to the receiver plane; and a programmable system configured to actuate the light source.

Another example useful for understanding the present invention can be an imaging method including: positioning a probe head adjacent a tissue, the probe head including: a light source positioned by the probe head to emit light toward the tissue, the light source comprising at least two light emitting elements, each light emitting element comprising an emission plane, the light including at least one spectral waveband suitable to generate a photoacoustic response in the tissue; and an ultrasound receiver positioned by the probe head to receive photoacoustic energy from the tissue, the ultrasound receiver comprising a receiver plane, wherein each emission plane is positioned at an acute angle with respect to the receiver plane; and actuating the light source.

Further areas of applicability of the present invention will become apparent from the detailed description provided hereinafter. It should be understood that the detailed description and specific examples, while indicating the preferred embodiment of the invention, are intended for purposes of illustration only and are not intended to limit the scope of the invention.

### BRIEF DESCRIPTION OF THE DRAWINGS

The foregoing summary, as well as the following detailed description of the exemplary embodiments, will be better understood when read in conjunction with the appended drawings. It should be understood, however, that the invention is not limited to the precise arrangements and instrumentalities shown in the following figures. Embodiments which do not fall within the scope of the claims do not constitute part of the invention, but are merely examples helpful for understanding the invention.
Figure 1 illustrates an imaging system in accordance with a first embodiment of the present invention;
Figure 2 illustrates the face of a probe head for the imaging system of FIG. 1;
Figure 3 illustrates a cross sectional view of the probe head for the imaging system of FIG. 1;
Figure 4 is a graph illustrating extinction coefficient for different substances versus wavelength;
Figures 5A-B illustrate cross sectional views of a probe head for alternative embodiments of the present invention;
Figure 6 illustrates a cross sectional view of a probe head for an alternative embodiment of the present invention;
Figure 7 schematically illustrates the imaging system of FIG. 1,
Figure 8 is a flowchart showing an imaging process for use with the imaging system of FIG. 7;
Figure 9 illustrates a cross sectional view of the probe head for an alternative embodiment of the present invention; and
Figure 10 illustrates a cross sectional view of a probe head for an alternative embodiment of the present invention.

### DETAILED DESCRIPTION

The following description of the preferred embodiment(s) is merely exemplary in nature and is in no way intended to limit the invention, its application, or uses.

The description of illustrative embodiments according to principles of the present invention is intended to be read in connection with the accompanying drawings, which are to be considered part of the entire written description. In the description of embodiments of the invention disclosed herein, any reference to direction or orientation is merely intended for convenience of description and is not intended in any way to limit the scope of the present invention. Relative terms such as "lower," "upper," "horizontal," "vertical," "above," "below," "up," "down," "left," "right," "top" and "bottom" as well as derivatives thereof (e.g., "horizontally," "downwardly," "upwardly," etc.) should be construed to refer to the orientation as then described or as shown in the drawing under discussion. These relative terms are for convenience of description only and do not require that the apparatus be constructed or operated in a particular orientation unless explicitly indicated as such. Terms such as "attached," "affixed," "connected," "coupled," "interconnected," and similar refer to a relationship wherein structures are secured or attached to one another either directly or indirectly through intervening structures, as well as both movable or rigid attachments or relationships, unless expressly described otherwise. Moreover, the features and benefits of the invention are illustrated by reference to the preferred embodiments. Accordingly, the invention expressly should not be limited to such preferred embodiments illustrating some possible non-limiting combinations of features that may exist alone or in other combinations of features; the scope of the invention being defined by the claims appended hereto.

Features of the present invention may be implemented in software, hardware, firmware, or combinations thereof. The programmable processes described herein are not limited to any particular embodiment, and may be implemented in an operating system, application program, foreground or background processes, driver, or any combination thereof. The computer programmable processes may be executed on a single processor or on or across multiple processors.

Processors described herein may be any central processing unit (CPU), microprocessor, micro-controller, computational, or programmable device or circuit configured for executing computer program instructions (e.g. code). Various processors may be embodied in computer and/or server hardware and/or computing device of any suitable type (e.g. desktop, laptop, notebook, tablet, cellular phone, smart phone, PDA, etc.) and may include all the usual ancillary components necessary to form a functional data processing device including without limitation a bus, software and data storage such as volatile and non-volatile memory, input/output devices, a display screen, graphical user interfaces (GUIs), removable data storage, and wired and/or wireless communication interface devices including Wi-Fi, Bluetooth, LAN, etc.

Computer-executable instructions or programs (e.g. software or code) and data described herein may be programmed into and tangibly embodied in a non-transitory computer-readable medium that is accessible to and retrievable by a respective processor as described herein which configures and directs the processor to perform the desired functions and processes by executing the instructions encoded in the medium. A device embodying a programmable processor configured to such non-transitory computer-executable instructions or programs is referred to hereinafter as a "programmable device", or just a "device" for short, and multiple programmable devices in mutual communication is referred to as a "programmable system". It should be noted that non-transitory "computer-readable medium" as described herein may include, without limitation, any suitable volatile or non-volatile memory including random access memory (RAM) and various types thereof, read-only memory (ROM) and various types thereof, USB flash memory, and magnetic or optical data storage devices (e.g. internal/external hard disks, floppy discs, magnetic tape CD-ROM, DVD-ROM, optical disk, ZIP^{™} drive, Blu-ray disk, and others), which may be written to and/or read by a processor operably connected to the medium.

In certain embodiments, the present invention may be embodied in the form of computer-implemented processes and apparatuses such as processor-based data processing and communication systems or computer systems for practicing those processes. The present invention may also be embodied in the form of software or computer program code embodied in a non-transitory computer-readable storage medium, which when loaded into and executed by the data processing and communications systems or computer systems, the computer program code segments configure the processor to create specific logic circuits configured for implementing the processes.

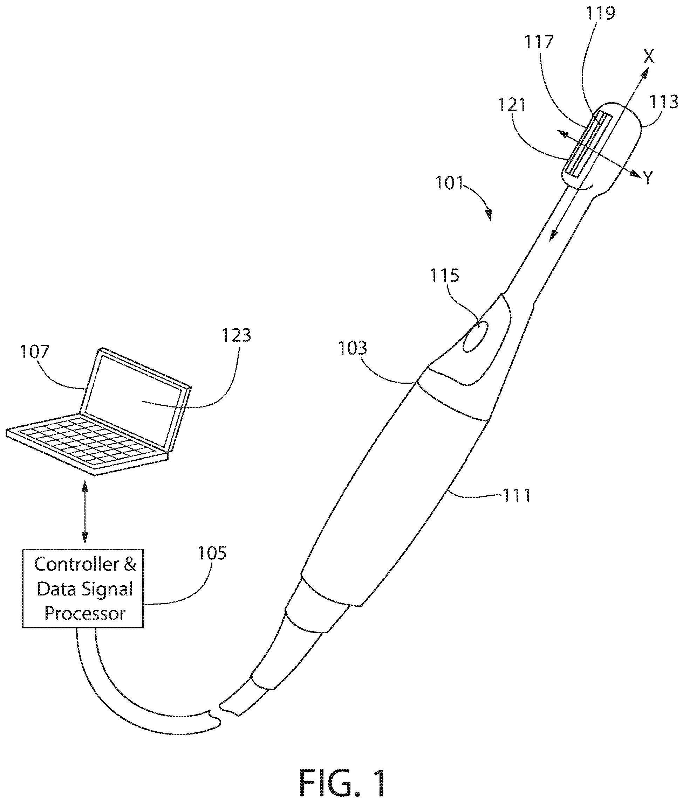

Turning in detail to the drawings, FIG. 1 illustrates an imaging system 101 in accordance with an embodiment of the present invention. The imaging system 101 includes a probe 103 which is operationally coupled to a controller and data signal processor 105 and to a computing device 107. The probe 103 includes a probe handle 111 coupled to a probe head 113. The probe handle 111 and the probe head 113 extend longitudinally along an x-axis.

The probe body 111 includes a button 115 which electronically actuates operation of the probe 103. The button 115 is electronically coupled to the controller and data signal processor 105, and when the button 115 is pressed, the controller and data signal processor 105 begins the data acquisition process. When the button 115 is released, the controller and data signal processor 105 terminates the data acquisition process. In certain embodiments, the button 115 may be a dual action button, such that a first press begins the data acquisition process, and a second press terminates the data acquisition process. In still other embodiments, the button 115 may be replaced by a switch or any other type of device which accepts user input to control operation of the probe 103.

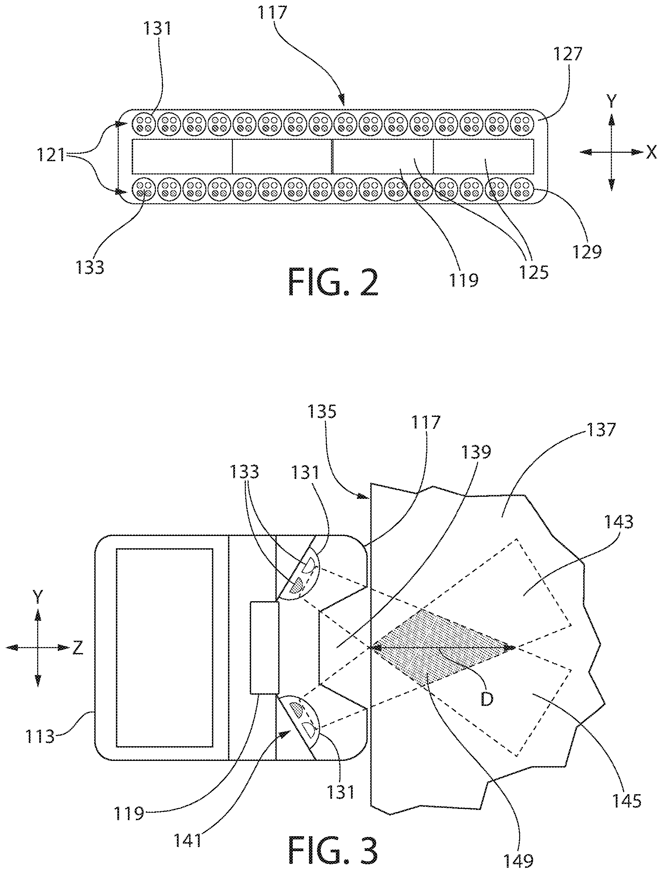

The probe head 113 includes a probe face 117 which is positioned adjacent tissue that is to be imaged. As shown in greater detail in FIG. 2, the probe face 117 includes an ultrasound element 119 and a light source 121, with the light source 121 being formed by two rows 127, 129 of light emitting elements 131 positioned on opposite sides of the ultrasound element 119. Each row 127, 129 of light emitting elements 131 forms an array which is operated as a single unit. In certain embodiments, however, the light emitting elements 131 may each be operated independently from one another. The ultrasound element 119 is formed by four linear array transducers 125 arranged end-to-end to form a transducer plane extending parallel to the plane formed by the x- and y-axes. As shown, the transducer plane is in the plane of the drawing. In certain embodiments, more or fewer of the linear array transducers 125 may be used. In certain embodiments, the linear array transducers 125 may each be a variable frequency ultrasound transducer configured to operate in the 40 MHz to 80 MHz range, with each having 64 transducer elements, such that the ultrasound element 119 is configured with a total of 256 channels. The invention, however, is not to be limited by the type of ultrasound transducer unless expressly stated in the claims.

Each light emitting element 131 may include one or more laser diodes (LDs) or light emitting diodes (LEDs), such that each light emitting element 131 may emit light in at least one spectral waveband suitable to generate a photoacoustic response in tissue. For simplification, the following description will use LDs as the light emitting elements 131, with the understanding that and LED may also be used. As shown, each light emitting element 131 includes a plurality of LDs 133 so that each can emit light in several different spectral wavebands. Each light emitting element 131 includes four LDs 133, with each producing light in a different spectral waveband. As discussed in more detail below, the different spectral wavebands enable the imaging system 101 to measure the absorption of certain compounds within the tissue, thereby allowing for quantification of the presence of those compounds in a depth resolved manner.

In certain embodiments, each light emitting element 131 includes at least one LD, and emits light in at least one of a waveband in the visual spectrum and a waveband in the infrared spectrum. In certain other embodiments, each light emitting element 131 includes at least two LDs, and emits light in one waveband in the visual spectrum and in one waveband in the infrared spectrum. In still other embodiments, each light emitting element 131 includes four LDs, and emits light in one waveband in the visual spectrum and three distinct wavebands in the infrared spectrum. In yet other embodiments, the number of wavebands emitted by the light emitting elements 131 may vary from one to many, not to be limiting of the invention unless otherwise expressly indicated in the claims.

The computing device 107 includes a display screen 123 for displaying images of the tissue. The images may be ultrasound B-mode and/or Doppler images, and/or the images may be photoacoustic images. The images may be displayed on the display screen 123 individually, or two or more of the image modalities may be displayed co-registered.

A cross-sectional view of the probe head 113 is shown in FIG. 3, with the probe face 117 placed against the surface 135 of tissue 137. In general, the tissue 137 will be *in vivo*, although the invention is not to be so limited. A channel 139 is positioned between the ultrasound transducer 119 and the surface 135 of the tissue 137. During use, the channel 139 may be filled with a coupling gel or other material that is transparent to both ultrasound energy and the spectral wavebands emitted by the light emitting elements 131. Filling the channel 139 with such a material serves to increase the coupling efficiency for the ultrasound energy between the ultrasound transducer 119 and the tissue 137. As discussed in greater detail below, the ultrasound transducer 119 produces ultrasound energy which is directed into the tissue 137, and the tissue reflects some of that ultrasound energy back to be detected by the transducer 119. Also, the spectral wavebands produced by the light emitting elements may be selected to produce photoacoustic energy due to the presence or one or more types of compounds within the tissue that absorb the selected wavebands.

The light emitting elements 131 form two rows 127, 129 located on opposite sides of the ultrasound transducer 119, with each light emitting element 131 positioned on an emission plane 141. Each light emitting element 131 directs light away from the emission plane 141 and into the tissue 137. The emission planes 141 for all the light emitting elements 131 in each of the individual rows 127, 129 are coplanar. In certain embodiments, the emission plane 141 for each light emitting element 131 may be defined by a polycarbonate board to which the LDs 133 are mounted. In other embodiments, the emission planes 141 may be defined by other structure within the probe head 113. In still other embodiments, each emission plane 141 may be an imaginary plane defined within the probe head 113 by the respective positions of the LDs 133 of each light emitting element 131. Each emission plane 141 is positioned at an acute angle with respect to the transducer plane, such that the light cones 143, 145 produced by each light emitting element 131 are directed into the tissue 137 to form an imaging zone 149. As used herein, an acute angle is a non-zero angle. The depth D of the imaging zone 149 determines the depth of the photoacoustic image produced by the imaging system 101. Depending on the angle formed between the emission plane 141 and the transducer plane, the depth D of the imaging zone may vary between about 2 mm to 20 mm. In certain embodiments, each light emitting element 131 may include light beam shaping optics to shape the light cones 143, 145 and achieve a more uniform delivery of multiple wavebands of light from multiple LDs onto and in the tissue.

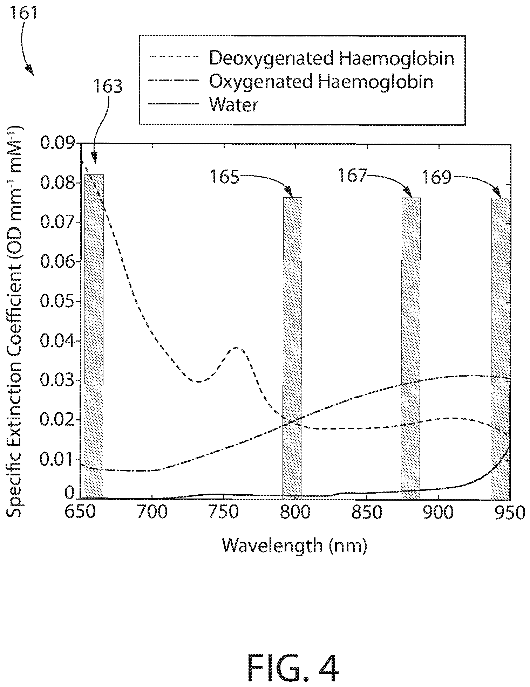

The graph 161 of FIG. 4 illustrates four useful wavebands within the visual and infrared spectrum that may be emitted by the light emitting elements 131. The graph 161 plots the specific extinction coefficient against wavelength for three different compounds: water, oxygenated hemoglobin, and deoxygenated hemoglobin. As can be seen in the graph, because the absorbance of light by water in the wavelength range shown is relatively low, the absorbance of light by the other compounds may be advantageously used to produce images of their distribution within tissue. The graph 161 also shows four wavebands that may be selected to aid in producing a photoacoustic image which advantageously can aid in depicting the presence of oxygenated hemoglobin and deoxygenated hemoglobin in the tissue. These wavebands include a first waveband 163 in the visual spectrum, centered near 650 nm, a second waveband 165 in the near infrared spectrum, centered near 805 nm, a third waveband 167 in the near infrared spectrum, centered near 860 nm, and a fourth waveband 169 in the near infrared spectrum, centered near 960 nm. The photoacoustic images may also be used to quantitatively depict the presence of other compounds, such as, without limitation, lipids and collagen, by selection of appropriate wavebands for light emitted from the light emitting elements 131. In certain embodiments, the wavebands are selected to be isolated absorption peaks or points of absorption curve separation for a compound of interest, such that the compound of interest may be measured without significant interference from absorption by other compounds that may be present. In certain embodiments, at least one of the wavebands emitted by the light emitting elements 131 may be selected as an isobastic point for compounds of interest, which in the graph 161 is the second waveband 165.Those of skill in the art will appreciate that the wavebands emitted by the light emitting elements 131 are based upon the specific LDs incorporated into the light emitting elements, such that the wavebands may be changed through the use of different LDs.

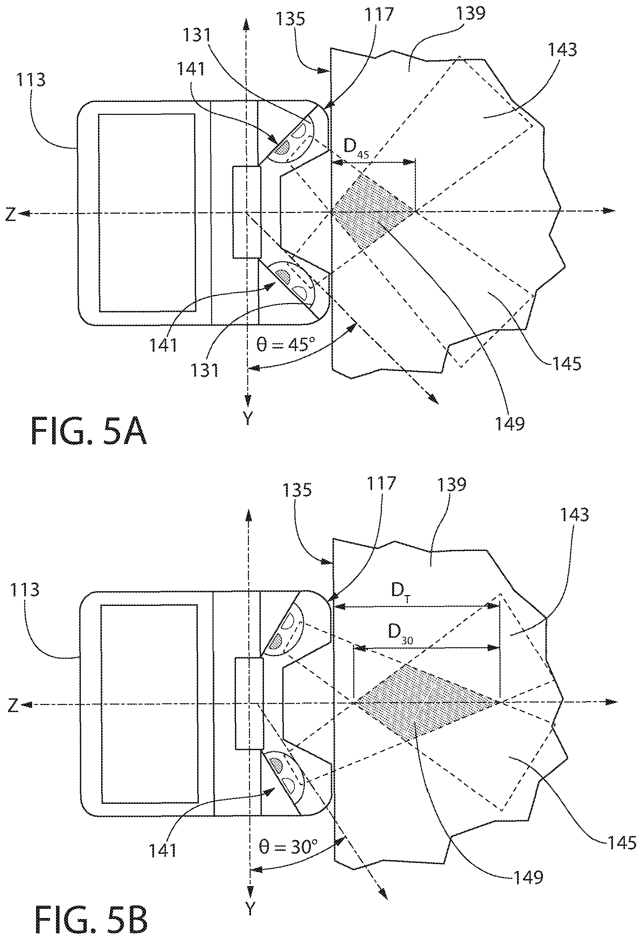

FIGS. 5A-B illustrate two different configurations for the probe head 113, each of which produce photoacoustic images having different depths of resolution within the tissue 139. As shown in FIG. 5A, the probe face 117 is placed against the surface 135 of the tissue 139. The light emitting elements 131 are positioned such that the respective emission planes 141 are placed at an angle θ of 45° with respect to the y-axis, and thus also with respect to the transducer plane. Having the angle of the emission planes 141 at θ = 45° with respect to the transducer plane results in the light cones 143, 145 forming an imaging zone 149 which has a depth of D₄₅ from the surface 135 of the tissue 139. By way of comparison, as shown in FIG. 5B, the light emitting elements 131 are positioned such that the respective emission planes 141 are placed at an angle 8 of 30° with respect to the y-axis, and thus also with respect to the transducer plane. Having the angle of the emission planes 141 at 8 = 30° with respect to the transducer plane results in the light cones 143, 145 forming an imaging zone 149 which has a depth of D₃₀ within the tissue 139, with the imaging zone extending a disstance of D_{T} from the surface 135 of the tissue 139, where D_{T} > D₃₀. In addition, as can be seen from FIGS. 5A-B, D₄₅ < D₃₀.

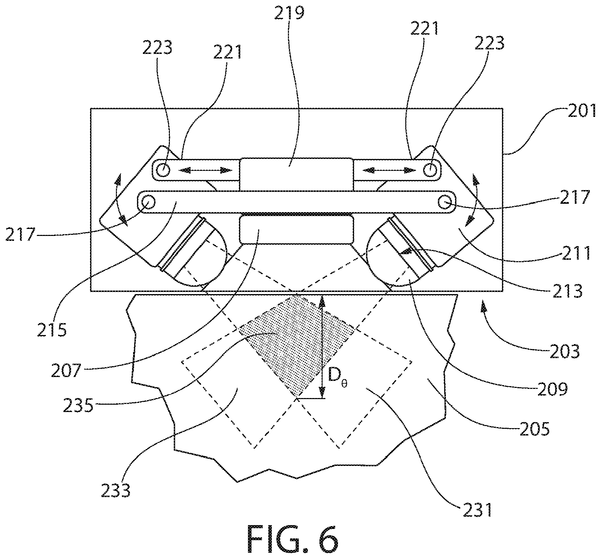

An alternative embodiment of a probe head 201 is shown in FIG. 6. This probe head 201 includes a probe face 203 which is placed against the tissue 205 such that the transducer 207 is positioned with respect to the tissue in the manner described above. The light emitting elements 209 are again positioned on opposite sides of the transducer 207. Each light emitting element 209 is affixed to a support structure 211 and emits light away from an emission plane 213 toward the tissue 205. Each support structure 211 is coupled to a support arm 215 at a pivot point 217, and the support arm 215 maintains each support structure 211 in a fixed translational position within the probe head 201. Each support structure 211 is also coupled to a squiggle motor 219 through a pivot arm 221 at a second pivot point 223. The squiggle motor 219 moves the pivot arms 221 laterally, and the lateral movement of the pivot arms 221 pivots the support structures 211 about their respective pivot points 217. Through operation of the squiggle motor 219, the angle between the emission planes 213 and the transducer plane may be altered before or during operation, such that the imaging zone 235 formed by the light cones 231, 233 has an angle-dependent depth of D_{θ} within the tissue. In certain embodiments, the squiggle motor 219 may be replaced with any other type of micro-mechanical device which is capable of creating controlling the pivot position of the support structures 211 for the light emitting elements 209. In certain embodiments, the controller associated with the imaging system may be used to provide control signals to the squiggle motor 219 so that the emission planes 213 may be placed at a desired angle with respect to the transducer plane.

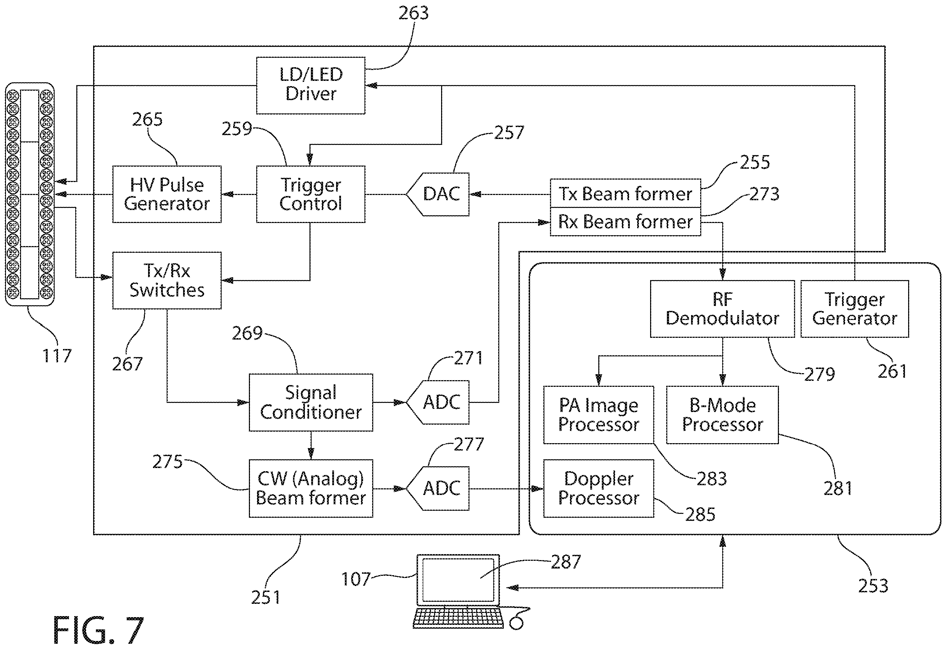

A schematic diagram of the imaging system 101 is illustrated in FIG. 7. A computing device 107 is operationally coupled to a data acquisition module 251 and to a control and image processing module 253. The computing device 107 may be any appropriate type of programmable device, such as a desktop or laptop computer, a tablet computer, or in some embodiments a smart phone. The computing device 107 may be programmed to control the operational parameters of the control functions, the data acquisition process, and the image processing. In addition, the computing device 107 includes a display screen 287 on which images produced by the imaging system 101 may be displayed.

The data acquisition module 251 is coupled to the probe face 117 to control of the ultrasound transducer and the light source and operates in a similar manner as compared to existing ultrasound imaging systems and existing photoacoustic imaging systems. However, the integration of the two systems does result in some important differences from known systems, and those differences are described herein. The data acquisition module 251 includes a transmit beam former 255 which generates the signal to be transmitted by the ultrasound transducer. The signal from the transmit beam former 255 passes through a digital to analogue converter 257 to a trigger control 259. The control and image processing module 253 includes a trigger generator 261 which generates a timing signal for actuating the light source on and off and for actuating the ultrasound transducer between a transmit mode and a receive mode. Through the use of a single timing signal, actuation of the light source and the ultrasound transducer can be used in tandem to gather both ultrasound data and photoacoustic data. The timing signal thus triggers the data acquisition module 251 to switch between an ultrasound mode and a photoacoustic mode. In the ultrasound mode, the data acquisition module 251 acquires data that is used to produce one or both of B-mode or Doppler ultrasound images, and in the photoacoustic mode, the data acquisition module 251 acquires data that is used to produce photoacoustic images. As indicated above, in the ultrasound mode, the data acquisition module 251 may generate ultrasound data in much the same way as a traditional ultrasound imaging system, and in the photoacoustic mode, the data acquisition module 251 may generate photoacoustic data in much the same way as a traditional photoacoustic imaging system.

In certain embodiments, the timing signal is configured to actuate the light source to emit thirty light pulses per second. By having thirty light pulses per second, the imaging system 101 is able to directly translate the images produced into a video having thirty frames per second (30 fps). In certain embodiments, the timing signal may be configured to actuate the light source to emit more or fewer than 30 pulses per second. The timing signal and the number of pulses emitted by the light source, however, are not to be limiting of the invention unless otherwise stated in the claims.

The timing signal from the trigger generator 261 is provided to the trigger control 259 and the LD driver 263. In response to the timing signal, the LD driver 263 controls the on and off state of the light source. Similarly, in response to the timing signal, the trigger control 259 controls the transmit and receive modes of the transducer. During the transmit mode, the trigger control 259 passes the converted signal generated by the transmit beam former 255 to the high voltage pulse generator 265, which in turn drives the transducer to generate the ultrasound energy directed into the tissue.

The timing signal from the trigger generator 261 is also provided to the transmit/receive switches 267, which includes one switch per ultrasound transducer element. The transmit/receive switches 267 control when a data signal received by the transducer is passed on for further processing by the signal conditioner 269. The transmit/receive switches 267 thus act as gate for data signals generated by the transducer, thereby effectively placing the transducer into a receive mode when the transmit/receive switches 267 enable the transducer signal to pass.

The signal conditioner 269 conditions the data signals received from the transducer for further processing. One of the issues that arise from the transducer sensing both reflected ultrasound energy and photoacoustic energy is that these two different types of energy can result in two different types of data signals being generated by the transducer. The data signal resulting from ultrasound energy will generally have a much higher voltage than the data signal resulting from photoacoustic energy. One purpose of the signal conditioner 269, therefore, is to normalize voltage levels of the data signals from the transducer so that the image processing module 253 can more easily process the two different types of data signals without having to have different circuits for each. The signal conditioner 269 also serves to protect down-circuit elements, which are designed to process the lower voltage photoacoustic data signals, from the higher voltage ultrasound data signals.

Data signals are passed from the signal conditioner 269 to an analogue to digital converter 271 and then to the receive beam former 273. The receive beam former 273 uses the data signal as feedback to help shape the signal generated by the transmit beam former 255. The data signal then passes into the image processing module 253, where it is processed first by the RF demodulator 279 and then by the photoacoustic image processor 283 or by the B-mode processor 281, as appropriate depending on whether the source of the data signal is ultrasound energy or photoacoustic energy. The source of the data signal may be determined based on the timing signal from the trigger generator 281.

The conditioned data signal from the signal conditioner 269 is also passed to the continuous wave (CW) beam former 275, which helps process the analog data signal for eventually producing Doppler images. From the CW beam former 275, the data signal is passed to another analogue to digital converter 277, and then into the image processing module 253, wherein it is processed by the Doppler processor 285 to produce Doppler images. As previously indicated, the different types of images (photoacoustic, B-mode, and Doppler) produced by the image processing module 253 are then communicated to the programmable device 107 for display on the display screen 287.

In certain embodiments, image processing may be performed solely within the image processing module 253, so that the programmable device 107 receives fully formed images and/or video for display. In certain other embodiments, aspects of image processing may be distributed between the image processing module 253 and the programmable device 107. In still other embodiments, the image processing module 253 may be incorporated into the programmable device 107 such that the entirety to of the image processing is performed by the programmable device 107.

In certain embodiments, the image processing system of FIG. 7 may be used with one of the photoacoustic acquisition subsystem or the ultrasound acquisition subsystem disabled. In such embodiments, the image processing system would perform nearly identically to a traditional photoacoustic imaging system or a traditional ultrasound imaging system, respectively. Such selection of one of the image acquisition modalities absent the other may be provided as a selectable option through the programmable device 107.

The different types of images may be displayed individually on the display screen, or one or more of the image types may be displayed overlapped with co-registration. Displaying the co-registered images often aids in providing additional contextual information which is unavailable from viewing the images individually or even side-by-side. Co-registration, therefore, may provide significant advantages in the clinical setting.

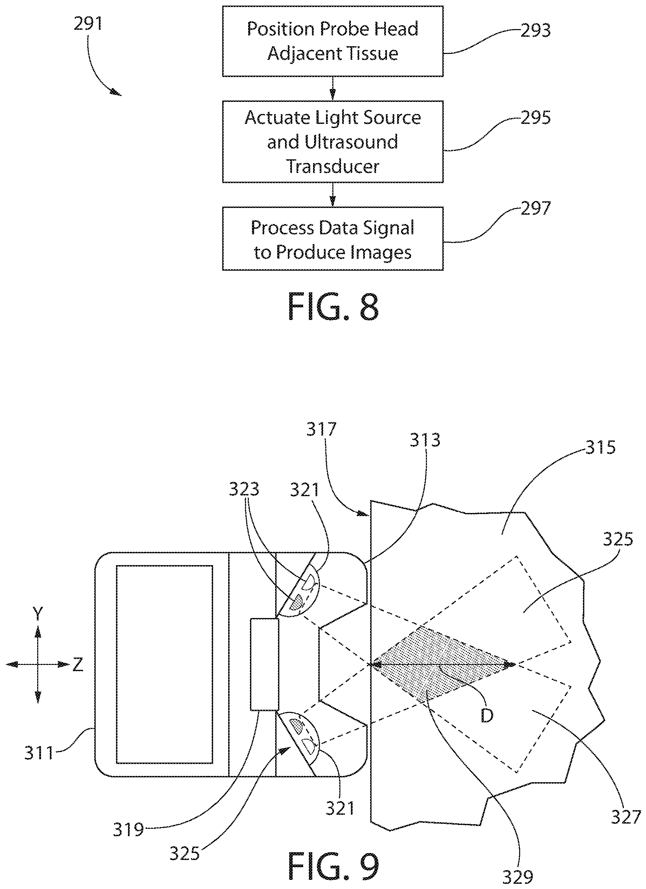

FIG. 8 shows a flowchart 291 illustrating the data acquisition process using the imaging system 101 shown in FIG. 7. The first step 293 of the process is to position the probe head adjacent the tissue to be imaged. As indicated above, a coupling agent or material may be used in conjunction with the probe head to increase the coupling efficiency of ultrasound energy passing between the tissue and the probe head. Any such coupling agent also should be transparent to the wavebands generated by the light source to avoid interfering with the photoacoustic process. With the probe head in position adjacent the tissue, the next step 295 is to actuate the light source and the ultrasound transducer. As described above, actuation of the light source and the ultrasound transducer is accomplished using an appropriate timing signal so that both ultrasound data and photoacoustic data may be collected in tandem by the transducer. As the light source and the ultrasound transducer are being actuated, a data signal is generated and then processed as the last step 297 of the flowchart 291. One or more of a photoacoustic image, an ultrasound B-mode image, and an ultrasound Doppler image may be produced from the data signal generated by the transducer.

An alternative configuration for the probe head 311 is illustrated in FIG. 9. As shown, this probe head 311 has a probe face 313 which is placed against the surface 317 of tissue 315 so that images of the tissue 315 may be obtained. The probe head 311 includes an ultrasound receiver 319, such that the probe head 311 is configured to generate a data signal based only upon photoacoustic energy. In certain embodiments, the ultrasound receiver 319 may be an ultrasound transducer which is used solely in the receive mode. In certain other embodiments, the ultrasound receiver 319 may be an ultrasound transducer which is fully implemented in the circuitry, as described above in connection with FIG. 7, with the ultrasound acquisition portion of the system deactivated. In still other embodiments, the ultrasound receiver 407 may be an ultrasound transducer which is used both in the receive and transmit modes.

The light emitting elements 321 are shown on opposite sides of the ultrasound receiver 319, with each light emitting element 321 positioned on an emission plane 325. Each light emitting element 321 directs light away from the emission plane 325 and into the tissue 315. In certain embodiments, the emission plane 325 for each light emitting element 321 may be defined by a polycarbonate board to which the LDs 323 are mounted. In other embodiments, the emission planes 325 may be defined by other structure within the probe head 311. In still other embodiments, each emission plane 325 may be an imaginary plane defined within the probe head 311 by the respective positions of the LDs 323 of each light emitting element 321. Each emission plane 325 is positioned at an acute angle with respect to the transducer plane (which is parallel to the x-y plane and normal to the z-axis), such that the light cones 325, 327 produced by each light emitting element 321 are directed into the tissue 315 to form an imaging zone 329. The depth D of the imaging zone 329 determines the depth of the photoacoustic image produced by the imaging system. In certain embodiments, each light emitting element 321 may include light beam shaping optics to shape the light cones 325, 327 and achieve a more uniform delivery of multiple wavebands of light from multiple LDs onto and in the tissue.

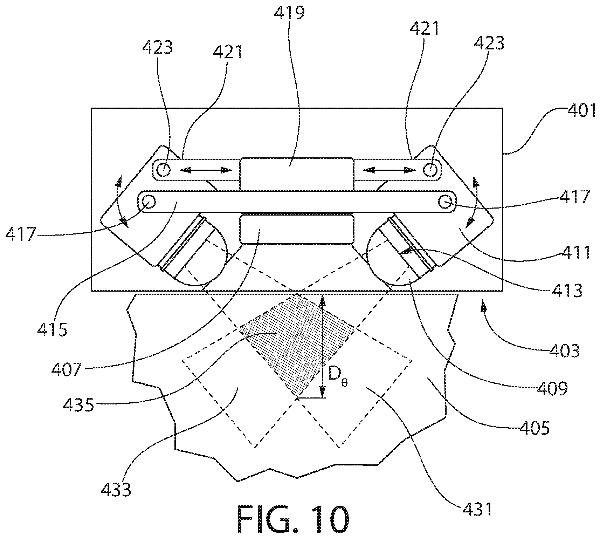

An alternative configuration for a probe head 401 is shown in FIG. 10. This probe head 401 also includes an ultrasound receiver 407 instead of an ultrasound transducer. In certain embodiments, the ultrasound receiver 407 may be an ultrasound transducer which is used solely in the receive mode. In still other embodiments, the ultrasound receiver 407 may be an ultrasound transducer which is used both in the receive and transmit modes. This probe head 401 includes a probe face 403 which is placed against the tissue 405 to position the ultrasound receiver 407 adjacent the tissue 405. The light emitting elements 409 are positioned on opposite sides of the ultrasound receiver 407. Each light emitting element 409 is affixed to a support structure 411 and emits light away from an emission plane 413 toward the tissue 405. Each support structure 411 is coupled to a support arm 415 at a pivot point 417, and the support arm 415 maintains each support structure 411 in a fixed translational position within the probe head 401. Each support structure 411 is also coupled to a squiggle motor 419 through a pivot arm 421 at a second pivot point 423. The squiggle motor 419 moves the pivot arms 421 laterally, and the lateral movement of the pivot arms 421 pivots the support structures 411 about their respective pivot points 417. Through operation of the squiggle motor 419, the angle between the emission planes 413 and the transducer plane may be altered before or during operation, such that the imaging zone 435 formed by the light cones 431, 433 has an angle-dependent depth of D_{θ} within the tissue. In certain embodiments, the squiggle motor 419 may be replaced with any other type of micro-mechanical device which is capable of creating controlling the pivot position of the support structures 411 for the light emitting elements 409. In certain embodiments, the controller associated with the imaging system may be used to provide control signals to the squiggle motor 419 so that the emission planes 413 may be placed at a desired angle with respect to the transducer plane. This embodiment provides a probe head 401 which may be used to produce photoacoustic images at varying depths within the tissue.

As used throughout, ranges are used as shorthand for describing each and every value that is within the range. Any value within the range can be selected as the terminus of the range. In addition, all references cited herein are hereby incorporated by referenced in their entireties. In the event of a conflict in a definition in the present disclosure and that of a cited reference, the present disclosure controls.

While the invention has been described with respect to specific examples including presently preferred modes of carrying out the invention, those skilled in the art will appreciate that there are numerous variations and permutations of the above described systems and techniques. It is to be understood that other embodiments may be utilized and structural and functional modifications may be made without departing from the scope of the present invention. Thus, the scope of the invention should be construed broadly as set forth in the appended claims.

## Claims

1. An imaging system (101) comprising:
a probe head (113) comprising:
a light source (121) for emitting light in at least one spectral waveband suitable to generate a photoacoustic response in tissue;
an ultrasound transducer (119) having a transmit mode for transmitting ultrasound energy into the tissue and a receive mode for receiving the ultrasound energy reflected by the tissue and photoacoustic energy from the tissue;
a probe handle (111) coupled to the probe head, the probe handle and the probe head extending longitudinally along an x-axis; and
a programmable system (107) configured to actuate the light source and to actuate the ultrasound transducer between the transmit mode and the receive mode in response to a timing signal;
wherein the probe head is configured to be insertable into an oral cavity of a human user to image oral tissues in the oral cavity, and
the ultrasound transducer comprises a linear array transducer (125) forming a transducer plane extending parallel to a plane containing the x-axis.

2. The imaging system of claim 1, wherein the at least one spectral waveband is in at least one of a visual spectrum and an infrared spectrum.

3. The imaging system of claim 2, wherein the at least one spectral waveband comprises a first waveband in the visual spectrum and a second waveband in the infrared spectrum.

4. The imaging system of any one of claims 1 to 3, wherein the light source comprises at least two light emitting elements positioned on opposite sides of the ultrasound transducer from one another.

5. The imaging system of any one of claims 1 to 4, wherein the programmable system receives a data signal generated by the ultrasound transducer and is programmed to produce from the data signal one or more of a photoacoustic image, an ultrasound B-mode image, and an ultrasound Doppler image.

6. The imaging system of claim 5, wherein the programmable system is programmed to display co-registered images from the one or more of the photoacoustic image, the ultrasound B-mode image, and the ultrasound Doppler image.

7. The imaging system of claim 5, wherein the programmable system is programmed to display a video comprising co-registered images from the one or more of the photoacoustic image, the ultrasound B-mode image, and the ultrasound Doppler image.

8. An imaging method comprising:
positioning a probe head (113) adjacent an oral tissue in an oral cavity of a human user, the probe head being coupled to a probe handle, the probe handle (111) and the probe head extending longitudinally along an x-axis, the probe head comprising:
a light source (121) positioned by the probe head to emit light toward the oral tissue, the light source including at least one spectral waveband suitable to generate a photoacoustic response in the oral tissue; and
an ultrasound transducer (119) positioned by the probe head to direct ultrasound energy into the tissue in a transmit mode and to receive the ultrasound energy reflected by the oral tissue and photoacoustic energy from the oral tissue in a receive mode, the ultrasound transducer comprising a linear array transducer (125) forming a transducer plane extending parallel to a plane containing the x-axis; and
actuating the light source and actuating the ultrasound transducer between the transmit mode and the receive mode in response to a timing signal.

9. The imaging method of claim 8, wherein the at least one spectral waveband is in at least one of a visual spectrum and an infrared spectrum.

10. The imaging method of claim 9, wherein the at least one spectral waveband comprises a first waveband in the visual spectrum and a second waveband in the infrared spectrum.

11. The imaging method of any one of claims 8 to 10, wherein the light source comprises at least two light emitting elements positioned on opposite sides of the ultrasound transducer from one another.

12. The imaging method of any one of claims 8 to 11, further comprising producing one or more of a photoacoustic image, an ultrasound B-mode image, and an ultrasound Doppler image from a data signal generated by the ultrasound transceiver.

13. The imaging method of claim 12, further comprising displaying the one or more of the photoacoustic image, the ultrasound B-mode image, and the ultrasound Doppler image as co-registered images.

## Patentansprüche

1. Abbildungssystem (101), umfassend:
einen Sondenkopf (113), umfassend:
eine Lichtquelle (121) zum Emittieren von Licht in mindestens einem spektralen Wellenband, der geeignet ist, eine photoakustische Reaktion im Gewebe zu erzeugen;
einen Ultraschallwandler (119) mit einem Sendemodus zum Senden von Ultraschallenergie in das Gewebe und einem Empfangsmodus zum Empfangen der vom Gewebe reflektierten Ultraschallenergie und photoakustischer Energie aus dem Gewebe;
einen mit dem Sondenkopf gekoppelten Sondengriff (111), wobei sich der Sondengriff und der Sondenkopf in Längsrichtung entlang einer x-Achse erstrecken; und
ein programmierbares System (107), das konfiguriert ist, um die Lichtquelle zu betätigen und den Ultraschallwandler zwischen dem Sendemodus und dem Empfangsmodus in Reaktion auf ein Zeitsignal zu betätigen;
wobei der Sondenkopf so konfiguriert ist, dass er in eine Mundhöhle eines menschlichen Benutzers eingeführt werden kann, um orale Gewebe in der Mundhöhle abzubilden, und
der Ultraschallwandler einen Linear-Array-Wandler (125) umfasst, der eine Wandlerebene bildet, die sich parallel zu einer Ebene erstreckt, die die x-Achse enthält.

2. Abbildungssystem nach Anspruch 1, wobei das mindestens eine spektrale Wellenband in mindestens einem von einem visuellen Spektrum und einem Infrarotspektrum liegt.

3. Abbildungssystem nach Anspruch 2, wobei das mindestens eine spektrale Wellenband ein erstes Wellenband im visuellen Spektrum und ein zweites Wellenband im Infrarotspektrum umfasst.

4. Abbildungssystem nach einem der Ansprüche 1 bis 3, wobei die Lichtquelle mindestens zwei lichtemittierende Elemente umfasst, die auf einander gegenüberliegenden Seiten des Ultraschallwandlers angeordnet sind.

5. Abbildungssystem nach einem der Ansprüche 1 bis 4, wobei das programmierbare System ein vom Ultraschallwandler erzeugtes Datensignal empfängt und so programmiert ist, dass es aus dem Datensignal ein photoakustisches Bild, ein Ultraschall-B-Mode-Bild oder ein Ultraschall-Doppler-Bild oder mehrere davon erzeugt.

6. Abbildungssystem nach Anspruch 5, wobei das programmierbare System so programmiert ist, dass es co-registrierte Bilder aus dem photoakustischen Bild, dem Ultraschall-B-Mode-Bild oder dem Ultraschall-Doppler-Bild oder mehreren davon anzeigt.

7. Abbildungssystem nach Anspruch 5, wobei das programmierbare System so programmiert ist, dass es ein Video anzeigt, das ko-registrierte Bilder aus dem photoakustischen Bild, dem Ultraschall-B-Mode-Bild oder dem Ultraschall-Doppler-Bild oder mehreren davon umfasst.

8. Abbildungsverfahren, umfassend:
Positionieren eines Sondenkopfes (113) in der Nähe eines oralen Gewebes in einer Mundhöhle eines menschlichen Benutzers, wobei der Sondenkopf mit einem Sondengriff gekoppelt ist, wobei sich der Sondengriff (111) und der Sondenkopf in Längsrichtung entlang einer x-Achse erstrecken, wobei der Sondenkopf umfasst:
eine Lichtquelle (121), die durch den Sondenkopf positioniert ist, um Licht in Richtung des oralen Gewebes zu emittieren, wobei die Lichtquelle mindestens ein spektrales Wellenband umfasst, das geeignet ist, eine photoakustische Reaktion in dem oralen Gewebe zu erzeugen; und
einen Ultraschallwandler (119), der durch den Sondenkopf positioniert ist, um Ultraschallenergie in das Gewebe in einem Sendemodus zu leiten und die Ultraschallenergie, die durch das orale Gewebe reflektiert wird, und photoakustische Energie von dem oralen Gewebe in einem Empfangsmodus zu empfangen, wobei der Ultraschallwandler einen Linear-Array-Wandler (125) umfasst, der eine Wandlerebene bildet, die sich parallel zu einer Ebene erstreckt, die die x-Achse enthält; und
Betätigen der Lichtquelle und Betätigen des Ultraschallwandlers zwischen dem Sendemodus und dem Empfangsmodus in Reaktion auf ein Zeitsignal.

9. Abbildungsverfahren nach Anspruch 8, wobei das mindestens eine spektrale Wellenband in mindestens einem visuellen Spektrum oder einem Infrarotspektrum liegt.

10. Abbildungsverfahren nach Anspruch 9, wobei das mindestens eine spektrale Wellenband ein erstes Wellenband im visuellen Spektrum und ein zweites Wellenband im Infrarotspektrum umfasst.

11. Abbildungsverfahren nach einem der Ansprüche 8 bis 10, wobei die Lichtquelle mindestens zwei lichtemittierende Elemente umfasst, die auf einander gegenüberliegenden Seiten des Ultraschallwandlers angeordnet sind.

12. Abbildungsverfahren nach einem der Ansprüche 8 bis 11, ferner umfassend Erzeugen eines oder mehrerer aus einem photoakustischen Bild, einem Ultraschall-B-Mode-Bild und einem Ultraschall-Doppler-Bild aus einem von dem Ultraschall-Sendeempfänger erzeugten Datensignal.

13. Abbildungsverfahren nach Anspruch 12, ferner umfassend Anzeigen eines oder mehrerer des photoakustischen Bildes, des Ultraschall-B-Mode-Bildes oder des Ultraschall-Doppler-Bildes als ko-registrierte Bilder.

## Revendications

1. Système d'imagerie (101) comprenant :
une tête de sonde (113) comprenant :
une source de lumière (121) pour émettre de la lumière dans au moins une bande d'onde spectrale appropriée pour générer une réponse photoacoustique dans un tissu ;
un transducteur à ultrasons (119) ayant un mode de transmission pour transmettre de l'énergie ultrasonore dans le tissu et un mode de réception pour recevoir l'énergie ultrasonore réfléchie par le tissu et l'énergie photoacoustique provenant du tissu ;
une poignée de sonde (111) accouplée à la tête de sonde, la poignée de sonde et la tête de sonde s'étendant longitudinalement le long d'un axe x ; et
un système programmable (107) conçu pour actionner la source de lumière et pour actionner le transducteur à ultrasons entre le mode d'émission et le mode de réception en réponse à un signal de synchronisation ;
dans lequel la tête de sonde est conçue pour pouvoir être insérée dans la cavité buccale d'un utilisateur humain pour imager les tissus buccaux dans la cavité buccale, et
le transducteur à ultrasons comprend un transducteur à réseau linéaire (125) formant un plan de transducteur s'étendant parallèlement à un plan contenant l'axe x.

2. Système d'imagerie selon la revendication 1, dans lequel l'au moins une bande d'onde spectrale se situe dans un spectre visuel et/ou un spectre infrarouge.

3. Système d'imagerie selon la revendication 2, dans lequel l'au moins une bande d'onde spectrale comprend une première bande d'onde dans le spectre visuel et une seconde bande d'onde dans le spectre infrarouge.

4. Système d'imagerie selon l'une quelconque des revendications 1 à 3, dans lequel la source de lumière comprend au moins deux éléments électroluminescents positionnés sur les côtés opposés l'un de l'autre du transducteur à ultrasons.

5. Système d'imagerie selon l'une quelconque des revendications 1 à 4, dans lequel le système programmable reçoit un signal de données généré par le transducteur à ultrasons et est programmé pour produire à partir du signal de données une ou plusieurs images parmi une image photoacoustique, une image ultrasonore en mode B et une image échographique Doppler.

6. Système d'imagerie selon la revendication 5, dans lequel le système programmable est programmé pour afficher des images co-enregistrées à partir desdites une ou plusieurs images parmi l'image photoacoustique, l'image échographique en mode B et l'image échographique Doppler.

7. Système d'imagerie selon la revendication 5, dans lequel le système programmable est programmé pour afficher une vidéo comprenant des images co-enregistrées provenant de l'une ou plusieurs de l'image photoacoustique, de l'image échographique en mode B et de l'image échographique Doppler.

8. Procédé d'imagerie comprenant :
positionner une tête de sonde (113) adjacente à un tissu buccal dans la cavité buccale d'un utilisateur humain, la tête de sonde étant accouplée à une poignée de sonde, la poignée de sonde (111) et la tête de sonde s'étendant longitudinalement le long d'un axe x, la tête de sonde comprenant :
une source de lumière (121) positionnée par la tête de sonde pour émettre de la lumière vers le tissu buccal, la source de lumière comprenant au moins une bande d'onde spectrale appropriée pour générer une réponse photoacoustique dans le tissu buccal ; et
un transducteur à ultrasons (119) positionné par la tête de sonde pour diriger l'énergie ultrasonore dans le tissu dans un mode de transmission et pour recevoir l'énergie ultrasonore réfléchie par le tissu buccal et l'énergie photoacoustique provenant du tissu buccal dans un mode de réception, le transducteur à ultrasons comprenant un transducteur à réseau linéaire (125) formant un plan de transducteur s'étendant parallèlement à un plan contenant l'axe x ; et
actionner la source de lumière et actionner le transducteur à ultrasons entre le mode d'émission et le mode de réception en réponse à un signal de synchronisation.

9. Procédé d'imagerie selon la revendication 8, dans lequel l'au moins une bande d'onde spectrale se situe dans un spectre visuel et/ou un spectre infrarouge.

10. Procédé d'imagerie selon la revendication 9, dans lequel l'au moins une bande d'onde spectrale comprend une première bande d'onde dans le spectre visuel et une second e bande d'onde dans le spectre infrarouge.

11. Procédé d'imagerie selon l'une quelconque des revendications 8 à 10, dans lequel la source de lumière comprend au moins deux éléments électroluminescents positionnés sur des côtés opposés l'un de l'autre du transducteur à ultrasons.

12. Procédé d'imagerie selon l'une quelconque des revendications 8 à 11, comprenant en outre la production d'une ou plusieurs images parmi une image photoacoustique, une image échographique en mode B et une image échographique Doppler à partir d'un signal de données généré par l'émetteur-récepteur ultrasonore.

13. Procédé d'imagerie selon la revendication 12, comprenant en outre l'affichage desdites une ou plusieurs images parmi l'image photoacoustique, l'image échographique en mode B et l'image échographique Doppler en tant qu'images co-enregistrées.
